Europäisches Patentamt

European Patent Office

Office européen des brevets

(19)

(11) EP 0 661 077 B1

(12) FASCICULE DE BREVET EUROPEEN

(45) Date de publication et mention
de la délivrance du brevet:
**29.07.1998 Bulletin 1998/31**

(51) Int Cl.6: **H04B 5/00, A61N 1/372**

(21) Numéro de dépôt: **94403058.4**

(22) Date de dépôt: **30.12.1994**

(54) **Appareil médical extrayant un signal utile mélé à des signaux parasites**

Medizinische Vorrichtung, welche ein Nutzsignal aus Störsignalen herausfiltert

Medical apparatus extracting a wanted signal mixed with unwanted signals

(84) Etats contractants désignés:
**BE CH DE ES FR GB IT LI SE**

(30) Priorité: **31.12.1993 FR 9315940**

(43) Date de publication de la demande:
**05.07.1995 Bulletin 1995/27**

(73) Titulaire: **ELA MEDICAL (Société anonyme)
F-92541 Montrouge (FR)**

(72) Inventeurs:
• **Deschamps, Hervé
F-92150 Suresnes (FR)**
• **Lee, Chikyan
F-94110 Arcueil (FR)**

(74) Mandataire: **Dupuis-Latour, Dominique et al
Avocat à la Cour,
Cabinet Bardehle, Pagenberg & Partner,
45, avenue Montaigne
75008 Paris (FR)**

(56) Documents cités:
**FR-A- 2 465 474          US-A- 5 069 210**

## Description

La présente invention concerne le domaine des dispositifs médicaux implantés actifs, et plus particulièrement le recueil des signaux émis lors de séquences de communication entre le dispositif implanté et une console de contrôle extérieure.

Les dispositifs médicaux implantés actifs comprennent notamment les stimulateurs cardiaques, les défibrillateurs, les appareils neurologiques, les pompes de diffusion de substance médicale, et les implants cochléaires.

Ces appareils, une fois implantés, sont programmés de l'extérieur au moyen d'une console appelée "programmateur". La vérification des paramètres de l'implant ou la transmission d'informations enregistrées par celui-ci s'effectuent par voie électromagnétique, appelée "télémétrie" dans la technique en question. Cette console est munie d'un élément récepteur qui est placé en regard du site de l'implant. Cet élément comprend une bobine qui capte le champ magnétique en provenance de l'appareil implanté.

Cette bobine, qui est typiquement formée d'une quarantaine de spires enroulées autour d'une surface d'environ 25 cm$^2$, recueille un signal utile compris entre 10 µV et 1000 µV suivant la distance bobine-implant, ce qui correspond, aux fréquences utilisées (typiquement de l'ordre de 128 kHz), à un champ magnétique compris entre $1,25.10^{-10}$ et $125.10^{-10}$ T (1,25 et 125 microgauss).

Ce champ est extrêmement faible et le système de réception est sensible à des perturbations électromagnétiques du même ordre de grandeur. À titre d'exemple, un champ électrique de même fréquence et d'amplitude 40 à 4000 mV/m induit dans la bobine des signaux de même grandeur que le signal utile émis par l'implant. De tels signaux perturbateurs sont créés par les lignes électriques porteuses du courant domestique (à la fréquence du réseau) ou par des appareils électroniques tels que les consoles de visualisation des systèmes informatiques (à la fréquence de balayage ligne).

Pour éliminer ce bruit, plusieurs solutions ont été mises en oeuvre, de façon isolée ou combinée.

Le US-A-4 944 299 décrit à cet effet un système de filtrage associé à un système de codage de l'information afin de réduire les risques d'erreurs dues aux perturbations externes, le bruit étant éliminé du fait qu'il ne contient pas les caractéristiques du signal attendu. Ce traitement n'est cependant pas opéré au niveau de la réception du signal, mais en aval, après détection.

Le DE-A-39 36 547 apporte une solution en amont du traitement du signal, par une élimination sélective du bruit recueilli. Le dispositif décrit comprend deux bobines dont l'une est placée directement en regard du signal émis par l'implant et l'autre est disposée par rapport à la première d'une façon telle qu'elle ne reçoive qu'une très faible partie de ce signal. En revanche, par un montage approprié, toutes deux reçoivent la même quantité de signaux perturbateurs en provenance de sources lointaines. L'utilisation de deux bobines identiques montées en opposition fait que ces signaux d'origine lointaine sont soustraits avant d'être présentés à la chaîne d'amplification. De cette façon, seule une faible partie du signal utile (source proche) est atténuée et la plus grande partie des signaux perturbateurs (source lointaine) est éliminée.

Toutefois ce système ne peut éliminer complètement un signal perturbateur dont la source d'émission n'est pas éloignée des deux bobines (source "semi-lointaine"). Une telle configuration est typiquement celle d'un moniteur de visualisation situé à une trentaine de centimètres de distance de la bobine de programmation. En effet, la différence des distances de chacune des deux bobines par rapport à la distance d'une source d'émission d'interférences électromagnétiques n'est alors pas négligeable, et le système éliminera incomplètement ces signaux parasites. Ainsi, le montage du DE-A-39 36 547 élimine parfaitement les signaux d'origine lointaine, n'atténue pratiquement pas les signaux d'origine proche de l'une des bobines, mais n'élimine que partiellement les signaux ayant leur source dans une zone intermédiaire.

L'un des buts de la présente invention est de proposer un dispositif électronique qui élimine les signaux perturbateurs d'origine aussi bien lointaine que semi-lointaine (c'est-à-dire situés dans une zone intermédiaire, de transition entre champ proche et champ lointain) tout en conservant les signaux utiles d'origine proche sans altération.

Un autre but de l'invention est de proposer un système qui s'adapte automatiquement et de façon optimale pour extraire le signal utile en présence de différentes sources de bruit, quelles que soient leurs distances par rapport au système et quelle que soit leur dépendance temporelle.

L'invention a pour objet un dispositif pour extraire un signal utile, émis par un appareil médical implanté, dans un signal constitué d'une onde électromagnétique comprenant ce signal utile mêlé à des signaux parasites. À cet effet, le dispositif comporte : une pluralité de collecteurs d'onde disposés en des points distincts, ces collecteurs d'onde étant sensibles à la composante magnétique de l'onde et la distance qui les sépare étant négligeable vis-à-vis de la longueur d'onde du signal; une pluralité d'amplificateurs associés chacun à un collecteur d'onde respectif, les gains de ces amplificateurs étant tous ajustables isolément ; des moyens sommateurs, recevant en entrée les signaux délivrés par les amplificateurs et délivrant en sortie un signal résultant ; des moyens pour évaluer une grandeur représentative du niveau du signal résultant ; des moyens de calcul, pour commander les gains des amplificateurs de manière à minimiser ladite grandeur dans une étape préalable en l'absence de signal utile et à maintenir les gains aux valeurs ainsi déterminées dans une étape subséquente en présence du signal utile. De préférence, le nombre de collecteurs d'onde est de deux.

La grandeur représentative peut notamment être la valeur efficace du signal résultant, ou une valeur proportionnelle à cette dernière.

Les moyens de calcul peuvent être des moyens de calcul linéaire aptes à résoudre un système d'équations linéaires où les gains des amplificateurs constituent les inconnues.

L'invention a également pour objet un procédé pour extraire un signal utile, émis par un appareil médical implanté, dans un signal constitué d'une d'onde électromagnétique comprenant ce signal utile mêlé à des signaux parasites, caractérisé par les étapes consistant à: (a) dans une étape préalable : cesser l'émission du signal utile par l'appareil implanté, recueillir les signaux parasites par une pluralité de collecteurs d'onde disposés en des points distincts et reliés chacun à un amplificateur associé, ces collecteurs d'onde étant sensibles à la composante magnétique de l'onde et la distance qui les sépare étant négligeable vis-à-vis de la longueur d'onde du signal, sommer les signaux délivrés par les amplificateurs, évaluer une grandeur représentative du niveau du signal résultant de cette sommation, et déterminer les gains des amplificateurs permettant de minimiser ladite grandeur; (b) dans une étape subséquente : faire émettre le signal utile par l'appareil implanté, et recueillir par ladite pluralité de collecteurs d'onde ce signal utile mêlé aux signaux parasites, les gains des amplificateurs restant réglés sur les valeurs déterminées lors de l'étape préalable.

Avantageusement, on exécute de façon répétitive les étapes (a) et (b), les gains des amplificateurs étant réévalués à chaque réexécution de l'étape (a).

L'étape de détermination des gains des amplificateurs à l'étape (a) peut notamment être effectuée par établissement d'un système linéaire de P équations à P+1 inconnues dont les inconnues sont les gains et les coefficients sont des coefficients de couplage entre champs et antennes, en fixant arbitrairement l'une des valeurs de gain et en déduisant les P autres valeurs de gain en fonction de cette dernière.

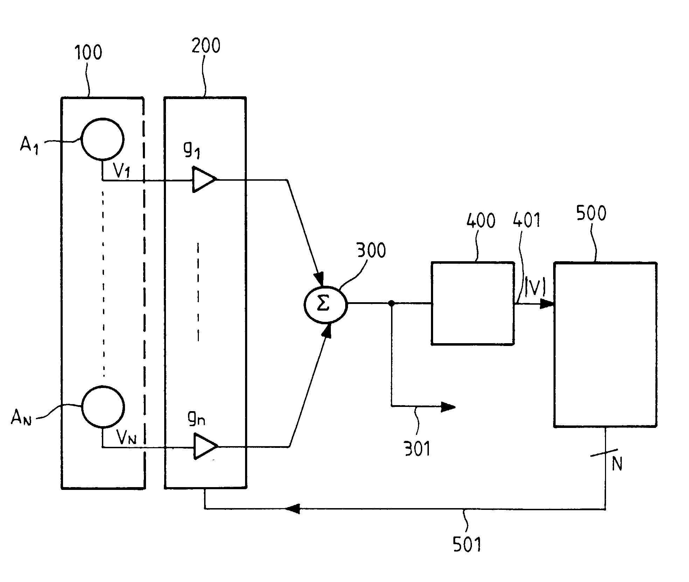

D'autres caractéristiques et avantages de l'invention apparaîtront à la lecture de la description détaillée ci-dessous d'un exemple de réalisation, faite en référence à la figure unique annexée, qui représente un schéma par blocs du dispositif de l'invention.

Dans le cas de la télémétrie des informations en provenance d'un implant, les signaux utiles ainsi que les signaux parasites sont véhiculés sous la forme de champs magnétiques. Le signal total $B_T$ (exprimé sous forme vectorielle) reçu au niveau d'un collecteur d'onde (en forme de bobine) se décompose à un instant $t$ donné, en tout point de l'espace donné dont $r$ est le vecteur position, en la somme du signal utile $B_s(r,t)$ et de P signaux parasites $B_i(r,t)$ dépendant de l'espace et du temps :

$$B_T(r,t) = B_s(r,t) + \sum_{i=1}^{P} B_i(r,t). \tag{1}$$

Si l'on utilise une seule bobine collectrice, on comprendra que celle-ci recevra tous les champs, signal et perturbations mêlés, sous la forme de $B_T(r,t)$.

L'idée de base de l'invention consiste à utiliser, au lieu d'une bobine collectrice unique, une pluralité de bobines, et à opérer une combinaison linéaire particulière des signaux délivrés par celles-ci permettant de conserver uniquement la composante $B_s$ du signal utile et d'éliminer les composantes $B_i$ en provenance des sources parasites.

Par hypothèse, on considérera que les diverses bobines sont situées en des points distincts, et que les composantes spectrales des signaux parasites significatives vis-à-vis de la bande passante du dispositif sont de longueur d'onde grande devant les dimensions du contenant enfermant les divers collecteurs d'onde. Cette dernière approximation signifie que, si deux bobines placées en deux points distincts recueillent le même champ parasite $B_i$, alors les signaux recueillis sont synchrones.

Dans l'application concernée, les longueurs d'onde des champs étudiés étant de l'ordre du kilomètre et les distances entre bobines de l'ordre de la dizaine de centimètres (limitation physique due à la taille d'un programmateur), l'approximation en question est justifiée.

De façon formelle, cette hypothèse permet d'écrire que les champs perturbateurs $B_i$ jouissent de la séparation des variables $r$ et $t$, et que l'on peut écrire, pour tout $i$:

$$B_i(r,t) = \lambda_i(r) \cdot \mu_i(t), \tag{2}$$

où $\lambda_i(r)$ est une fonction représentative de l'orientation et de l'amplitude du champ et $\mu_i(t)$ est une fonction exprimant

EP 0 661 077 B1

la dépendance temporelle de $B_i$.

Le dispositif de l'invention, illustré schématiquement sur la figure, comporte une pluralité 100 de bobines réceptrices $A_1$ à $A_N$, qui recueillent un flux magnétique induit et produisent des tensions $V_1$ à $V_N$ qui sont appliquées à une pluralité 200 d'amplificateurs respectifs, chacun avec un gain variable $g_1$ à $g_N$.

Plus précisément, au niveau de la $J^{ième}$ bobine de réception, la force électromotrice qui est créée est la somme de la dérivée des flux qui la traversent et s'exprime sous la forme :

$$V_j(t) = \sum_{i=1}^{P} C_{ij} * d\mu_i(t)/dt, \qquad (3)$$

où $C_{ij}$ est une constante de couplage entre le champ $B_i(r,t)$ et la $j^{ème}$ bobine.

Si l'on considère un ensemble de N bobines soumises à P champs parasites $B_i(r,t)$ dont les tensions $V_j(t)$ recueillies sur chaque bobine sont chacune amplifiées par un amplificateur de gain $g_j$, alors on peut montrer qu'il existe une combinaison de valeurs de gains $g_j$ telle que la somme des produits $V_j(t)*g_j$ s'annule, à la condition que N = P+1. En d'autres termes, tout ensemble de P signaux perturbateurs distincts peut avoir un effet annihilé si on les capte avec un nombre de bobines au moins supérieur d'une unité au nombre de signaux parasites. Une fois cet effet obtenu, la superposition du champ utile $B_s$ autorise sa détection de façon optimale dans la mesure où la somme des apports perturbateurs est annulée.

A cet effet, les gains des amplificateurs sont individuellement réglés par des signaux de commande 501 générés par un moyen de calcul 500.

Les tensions amplifiées sont sommées dans un moyen d'addition 300 pour fournir un signal résultant V. Un moyen de traitement 400 agissant sur V fournit une grandeur I V I 401 qui peut être une valeur efficace ou un module. Le moyen de calcul 500 évalue la grandeur I V I et ajuste individuellement les gains des amplificateurs 200, par des signaux de commande 501, de telle façon que la grandeur 401 soit minimale. Dans la mesure où le nombre de signaux perturbateurs est inférieur au nombre de bobines le système réglera les différents gains de telle façon que I V I soit nulle.

Cette phase d'annulation des perturbations étant réalisée, le signal utile peut être émis vers le réseau de bobines et donner lieu à un signal V non nul, qui sera utilisé à l'entrée d'un étage de traitement de son contenu.

En effet, ce signal ne peut être annulé par le système à N bobines, car les P=N-1 signaux perturbateurs sont déjà annulés et que, pour annuler aussi le signal utile, il faudrait N+1 bobines.

Une réalisation simplifiée de l'invention peut mettre en oeuvre un réseau de deux bobines identiques $A_1$ et $A_2$, seulement.

En effet, dans ce qui précède, on a raisonné sur P champs perturbateurs ayant chacun leur propre dépendance temporelle. Ceci signifie que P sources viennent perturber le recueil du signal utile, en émettant chacune leurs propres parasites (par exemple un moniteur vidéo qui émet une série de pics parasites correspondant aux retours de ligne, des conducteurs électriques qui émettent des signaux parasites à la fréquence du secteur, etc.).

Si l'on approche d'une source de perturbation, les parasites captés qui en proviennent vont augmenter en amplitude et le système sera très sensible à des déplacements. En revanche, si l'on est suffisamment loin des diverses sources, la forme du signal parasite total capté dépendra peu de la position du système des bobines collectrices et pourra être localement décrite par un champ unique. En d'autres termes, ceci signifie que si l'on ne bouge pas ou peu par rapport aux différentes sources perturbatrices en restant suffisamment éloigné de celles-ci, elles ne sont pas localisables et les signaux parasites reçus le seront comme s'ils provenaient d'une source perturbatrice unique, donc comme si l'on avait P=1. Deux bobines réceptrices sont alors suffisantes pour annuler ce champ perturbateur.

On va maintenant décrire la manière dont est mis en oeuvre le système de l'invention dans cette hypothèse simplifiée d'un système à deux bobines (la généralisation à un plus grand nombre de bobines étant immédiate).

Lors d'une première phase de fonctionnement du dispositif, une mesure de bruit ambiant est réalisé en l'absence de signal utile. Le calculateur 500 ajuste les valeurs des gains des deux amplificateurs de façon à minimiser la valeur de I V I. Dans cet exemple, on suppose donc que le champ perturbateur est assimilable à un champ unique $B_1(r,t)$. Toutefois, suivant la disposition des bobines par rapport à $B_1$, les tensions $V_1$ et $V_2$ peuvent ne pas être égales, et le réglage individuel des gains $g_1$ et $g_2$ permet d'annuler les effets non symétriques du champ $B_1$ sur chacune des bobines.

Une fois ce réglage préliminaire effectué, le signal utile $B_s$, émis dans une phase subséquente par le dispositif implanté, est reçu par le réseau des deux bobines et la valeur V correspondante, dépourvue de bruit, peut être envoyée en 301 vers un étage de détection (non représenté) en aval du dispositif pour traitement approprié. Par un choix avantageux de séquences alternant phases de recueil du bruit (phases de silence de l'implant) et phases d'émission du signal $B_s$, le système est capable de s'adapter automatiquement à des variations des effets au niveau des bobines du signal perturbateur causées par des déplacements de l'élément récepteur du programmateur ou éventuellement

4

de la source du bruit.

Il est clair que cette invention peut être réalisée par des moyens classiques électroniques, assistés ou non par des moyens logiciels. En particulier, le calculateur 500 peut avantageusement être constitué d'un module de calcul apte à résoudre un système d'équations linéaires, cet ensemble correspondant à l'annulation de la somme de produits $V_j(t){*}g_j$ pour tout $j \, \varepsilon \, [1,N]$ ; l'un des N gains sera fixé arbitrairement et tous les autres seront exprimés en fonction de celui-ci. Des indications détaillées sur la manière de résoudre un tel système d'équations linéaires pourront être trouvées dans C. Cagnac, E. Ramis et J. Commeau, *Traité de mathématiques spéciales*, tome I (*Algèbre*), chapitre XV-2 (*Systèmes de Kramer*), pages 123 et suivantes, éditions Masson, 1970.

**Revendications**

1. Un dispositif pour extraire un signal utile, émis par un appareil médical implanté, dans un signal constitué d'une onde électromagnétique comprenant ce signal utile mêlé à des signaux parasites, ainsi qu' une pluralité de collecteurs d'onde (100) disposés en des points distincts, ces collecteurs d'onde étant sensibles à la composante magnétique de l'onde et la distance qui les sépare étant négligeable vis-à-vis de la longueur d'onde du signal, caractérisé en ce qu'il comporte :

   – une pluralité d'amplificateurs (200) associés chacun à un collecteur d'onde respectif, les gains de ces amplificateurs ($g_1$ ... $g_N$) étant tous ajustables isolément,
   – des moyens sommateurs (300), recevant en entrée les signaux délivrés par les amplificateurs et délivrant en sortie un signal résultant ($V(t)$),
   – des moyens (400) pour évaluer une grandeur (IVI) représentative du niveau du signal résultant,
   – des moyens de calcul (500), pour commander les gains des amplificateurs de manière à minimiser ladite grandeur dans une étape préalable en l'absence de signal utile et à maintenir les gains aux valeurs ainsi déterminées dans une étape subséquente en présence du signal utile.

2. Le dispositif de la revendication 1, dans lequel le nombre de collecteurs d'onde est de deux.

3. Le dispositif de la revendication 1, dans lequel ladite grandeur représentative est la valeur efficace du signal résultant, ou une valeur proportionnelle à cette dernière.

4. Le dispositif de la revendication 1, dans lequel les moyens de calcul sont des moyens de calcul linéaire aptes à résoudre un système d'équations linéaires où les gains des amplificateurs constituent les inconnues.

5. Un procédé pour extraire un signal utile, émis par un appareil médical implanté, dans un signal constitué d'une onde électromagnétique comprenant ce signal utile mêlé à des signaux parasites, caractérisé par les étapes consistant à:

   a) dans une étape préalable:

   • cesser l'émission du signal utile par l'appareil implanté,
   • recueillir les signaux parasites par une pluralité de collecteurs d'onde disposés en des points distincts et reliés chacun à un amplificateur associé, ces collecteurs d'onde étant sensibles à la composante magnétique de l'onde et la distance qui les sépare étant négligeable vis-à-vis de la longueur d'onde du signal,
   • sommer les signaux délivrés par les amplificateurs,
   • évaluer une grandeur représentative du niveau du signai résultant de cette sommation, et
   • déterminer les gains des amplificateurs permettant de minimiser ladite grandeur,

   b) dans une étape subséquente :

   • faire émettre le signal utile par l'appareil implanté, et
   • recueillir par ladite pluralité de collecteurs d'onde ce signal utile mêlé aux signaux parasites, les gains des amplificateurs restant réglés sur les valeurs déterminées lors de l'étape préalable.

6. Le procédé de la revendication 5, dans lequel on exécute de façon répétitive les étapes (a) et (b), les gains des amplificateurs étant réévalués à chaque réexécution de l'étape (a).

**7.** Le procédé de la revendication 5, dans lequel l'étape de détermination des gains des amplificateurs à l'étape (a) est effectuée par établissement d'un système linéaire de P équations à P+1 inconnues dont les inconnues sont les gains et les coefficients sont des coefficients de couplage entre champs et antennes, en fixant arbitrairement l'une des valeurs de gain et en déduisant les P autres valeurs de gain en fonction de cette dernière.

**Patentansprüche**

**1.** Vorrichtung zum Extrahieren eines Nutzsignales, das durch einen medizinischen implantierten Apparat ausgesandt worden ist, aus einem Signal, das aus einer elektromagnetischen Welle gebildet ist, die dieses Nutzsignal gemischt mit parasitären Signalen aufweist, sowie eine Vielzahl von Wellensenken (100), die an unterschiedlichen Punkten angeordnet sind, wobei diese Wellensenken empfindlich bezüglich der magnetischen Komponente der Welle sind und wobei der Abstand, der sie voneinander trennt, vernachlässigbar bezüglich der Wellenlänge des Signales ist,
dadurch gekennzeichnet, daß sie aufweist:

- eine Vielzahl von Verstärkern (200), die jeweils einer Wellensenke zugeordnet sind, wobei die Gewinne dieser Verstärker ($g_1$ ... $g_N$) alle isoliert einstellbar sind,

- Summiereinrichtungen (300), die als Eingabe die Signale empfangen, die von den Verstärkern geliefert werden, und die als Ausgabe ein sich ergebendes Signal ($V(t)$) liefern,

- Einrichtungen (400), um eine Größe ($|V|$) zu evaluieren, die repräsentativ für das Niveau des sich ergebenden Signales ist,

- Recheneinrichtungen (500), um die Gewinne der Verstärker so zu steuern, daß die Größe in einem vorangehenden Schritt ohne das Nutzsignal minimiert wird und daß die Gewinne auf den so bestimmten Werten in einem darauffolgenden Schritt bei Vorhandensein des Nutzsignales gehalten werden.

**2.** Vorrichtung gemäß Anspruch 1, bei der die Anzahl von Wellensenken zwei ist.

**3.** Vorrichtung gemäß Anspruch 1, bei der die repräsentative Größe der quadratische Mittelwert des sich ergebenden Signales oder ein Wert ist, der proportional zu diesem letzteren ist.

**4.** Vorrichtung gemäß Anspruch 1, bei der die Recheneinrichtungen Einrichtungen zur linearen Berechnung sind, die dazu geeignet sind, ein lineares Gleichungssystem zu lösen, bei dem die Gewinne der Verstärker die Unbekannten bilden.

**5.** Verfahren, um ein Nutzsignal, das von einem medizinischen implantierten Apparat ausgesandt wird, aus einem Signal zu extrahieren, das aus einer elektromagnetischen Welle gebildet ist, die dieses Nutzsignal gemischt mit parasitären Signalen aufweist,
gekennzeichnet durch die Schritte, die darin bestehen:

a) in einem vorangehenden Schritt:

- die Emission des Nutzsignales durch den implantierten Apparat zu stoppen,

- die parasitären Signale durch eine Vielzahl von Wellensenken aufzunehmen, die an unterschiedlichen Punkten angeordnet sind und die jeweils mit einem zugeordneten Verstärker verbunden sind, wobei diese Wellensenken empfindlich für die magnetische Komponente der Welle sind und wobei der Abstand, der sie voneinander trennt, vernachlässigbar bezüglich der Wellenlänge des Signales ist,

- die durch die Verstärker gelieferten Signale aufzusummieren,

- eine Größe, die repräsentativ für das Niveau des sich aus dieser Summierung ergebenden Signal ist, zu evaluieren, und

- die Gewinne der Verstärker zu bestimmen, die erlauben, diese Größe zu minimieren,

b) in einem darauffolgenden Schritt:

- den implantierten Apparat das Nutzsignal aussenden zu lassen und

- dieses Nutzsignal, das mit den parasitären Signalen vermischt ist, durch die Vielzahl von Wellensenken aufzunehmen, wobei die Gewinne der Verstärker auf die bei dem vorangehenden Schritt bestimmten Werte eingestellt bleiben.

6. Verfahren gemäß Anspruch 5, bei dem man die Schritte (a) und (b) auf wiederholte Weise ausführt, wobei die Gewinne der Verstärker bei jeder Neuausführung des Schrittes (a) neu evaluiert werden.

7. Verfahren gemäß Anspruch 5, bei dem der Schritt der Bestimmung der Gewinne der Verstärker im Schritt (a) durch Erstellung eines linearen Systems von P Gleichungen mit P+1 Unbekannten ausgeführt wird, wobei die Unbekannten die Gewinne sind und wobei die Koeffizienten die Kopplungskoeffizienten zwischen den Feldern und Antennen sind, wobei einer der Gewinnwerte zufällig festgelegt wird und wobei die P anderen Gewinnwerte in Abhängigkeit von diesem letzteren abgeleitet werden.

## Claims

1. A device to extract useful signals, emitted by an implanted medical apparatus, from an electromagnetic wave signal including the useful signal mixed with parasitic signals, as well as a plurality of wave collectors (100) located at distinct points, each wave collector being sensitive to the magnetic component of the wave and the distance of separation between them being negligible relative to the wavelength of the signal, characterized in that it comprises

- a plurality of amplifiers (200), each amplifier being associated with one respective wave collector, the gains of these amplifiers ($g_1$ to $g_N$) being all adjustable in an isolated manner,

- summing means (300) receiving at the input the signals provided by the amplifiers and providing at its output a resulting signal (V($t$)),

- means (400) for evaluating a magnitude ( I V I ) representative of the level of the resulting signal,

- calculating means (500) for controlling the gains of the amplifiers in order to minimize the magnitude in a preliminary step in the absence of the useful signal, and to maintain the gains on the so determined values in a subsequent step in the presence of the useful signal.

2. The device of claim 1, in which the number of wave collectors is two.

3. The device of claim 1, in which the representative magnitude is the root mean square value of the resulting signal or a value proportional to the latter one.

4. The device of claim 1, in which the calculating means are linear calculating means able to solve a system of linear equations, the amplifier gains being the unknowns.

5. A process to extract an useful signal, emitted by an implanted medical apparatus, from an electromagnetic wave signal including the useful signal mixed with parasitic signals, characterized by the steps consisting in:

a) in a preliminary step:

- inhibiting the emission of the useful signal by the implanted apparatus,

- receiving the parasitic signals by a plurality of wave collectors located at different positions and respectively connected to an associated amplifier, the wave collectors being sensitive to the magnetic component of the wave and the distance separating them being negligible with respect to the wavelength of the signal,

- summing the signals provided by the amplifiers,

- evaluating a magnitude representative of the level of the signal resulting from this summing, and

- determining the amplifier gains allowing to minimize said magnitude,

b) in a subsequent step:

- making the implanted apparatus emit the useful signal, and

- receiving the useful signal mixed with parasitic signals by the plurality of wave collectors, the amplifier gains being controlled on the values determined at the preliminary step.

6. The method of claim 5, in which steps (a) and (b) are executed in a repeating manner, the amplifier gains being reevaluated at each re-execution of step (a).

7. The method of claim 5, in which the step of determining the amplifier gains in step (a) is effected by the establishment of a linear system of P equations with P+1 unknowns, the unknowns being the gains and the coefficients being the coupling coefficients between the fields and the antennas, one of the gain values being arbitrarily fixed and P other gain values being deducted depending on the latter one.